Europäisches Patentamt

European Patent Office

Office européen des brevets

(19)

(11) Veröffentlichungsnummer : **0 330 900 B1**

(12) **EUROPÄISCHE PATENTSCHRIFT**

(45) Veröffentlichungstag der Patentschrift :
**06.05.92 Patentblatt 92/19**

(51) Int. Cl.$^5$ : **A61M 5/14,** F16K 7/06

(21) Anmeldenummer : **89102477.0**

(22) Anmeldetag : **14.02.89**

(54) Schlauchklemme.

(30) Priorität : **01.03.88 DE 3806484**

(43) Veröffentlichungstag der Anmeldung :
**06.09.89 Patentblatt 89/36**

(45) Bekanntmachung des Hinweises auf die
Patenterteilung :
**06.05.92 Patentblatt 92/19**

(84) Benannte Vertragsstaaten :
**AT BE CH DE ES FR GB GR IT LI LU NL SE**

(56) Entgegenhaltungen :
**EP-A- 0 241 593**
**DE-A- 2 512 588**
**DE-A- 2 855 572**
**DE-B- 2 043 551**
**US-A- 4 270 725**
**US-A- 4 725 037**

(73) Patentinhaber : **B. Braun Melsungen AG**
**Carl-Braun Strasse**
**W-3508 Melsungen (DE)**

(72) Erfinder : **Lesemann, Egon**
**Lindenbergstrasse 28-30**
**W-3508 Melsungen (DE)**
Erfinder : **Maier, Hans-Otto, Dr.**
**Thüringer Strasse 7**
**W-3508 Melsungen (DE)**

(74) Vertreter : **Selting, Günther, Dipl.-Ing. et al**
**Patentanwälte von Kreisler, Selting, Werner**
**Deichmannhaus am Hauptbahnhof**
**W-5000 Köln 1 (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine Schlauchklemme nach dem Oberbegriff des Patentanspruchs 1.

Bei einer bekannten Schlauchklemme (DE-AS 20 43 551) ist im Kanalboden eine Ausweichnut mit V-förmigem Querschnitt vorgesehen, deren Tiefe sich in Längsrichtung des Kanals verändert. Der Schlauch ist im vollständig zusammengequetschten Zustand breiter als der Kanalboden, so daß ein Teil der Unterseite des Schlauchs von der Rolle in die Ausweichnut hinein verdrängt wird. Der Schlauch liegt dabei nur an den schrägen Rändern des Kanals auf. Obwohl durch eine derartige Ausbildung des Kanals das Relaxationsverhalten, d.h. das Rückformungsverhalten, des Schlauchs verbessert werden soll, indem Kaltfließen und ähnliche Ermüdungserscheinungen verhindert werden, erhält man keine definierte Konstanz der durch die Schlauchabquetschung hervorgerufenen Tropfrate, weil die Position, die der Schlauch im Inneren des Kanals und insbesondere in der Ausweichnut einnimmt, weitgehend von Zufälligkeiten abhängt.

US-A-4 270 725 beschreibt eine Schlauchklemme, bei der der Kanalboden eine längslaufende Ausweichnut von bogenförmigem Querschnitt aufweist. Hierbei ist eine sehr genaue Anpassung des Querschnitts von Kanal und Ausweichnut an den Querschnitt des Schlauchs erforderlich. Ist der Schlauchquerschnitt auch nur geringfügig zu groß, so erfolgen Stauchungen und Faltungen, mit der Folge, daß keine definierte Durchflußrate erzielbar ist.

Der Erfindung liegt die Aufgabe zugrunde, eine Schlauchklemme der im Oberbegriff des Patentanspruchs 1 angegebenen Art derart auszubilden, daß der Schlauch in definierter Weise von der Ausweichnut aufgenommen wird, so daß sich das Öffnungsvolumen im abgequetschten Zustand nicht verändert und die Durchflußrate konstant bleibt.

Die Lösung dieser Aufgabe erfolgt erfindungsgemäß mit den im kennzeichnenden Teil des Patentanspruchs 1 angegebenen Merkmalen.

Bei der erfindungsgemäßen Schlauchklemme wird der in die Ausweichnut gedrückte Teil des Schlauchquerschnitts durch eine Stützfläche am Boden der Ausweichnut abgestützt, so daß die Tiefe des ausweichenden Bereichs sogleich bei der Abstützung festgelegt wird und sich anschließend nicht mehr vergrößern kann. Damit ist keine kriechende Erweiterung des Öffnungsquerschnitts des Schlauchs in die Ausweichnut hinein möglich. Der anfangs eingestellte Öffnungsquerschnitt des Schlauchs bleibt über die Dauer der Einstellung konstant. Dadurch, daß die Breite der Stützfläche am breitesten Ende der Ausweichnut mindestens etwa die Hälfte der Nutbreite beträgt, wird sichergestellt, daß der ausweichende Bereich des Schlauchquerschnitts beim Abquetschen sogleich von der Stützfläche des Bodens der Ausweichnut abgestützt wird und danach keine Vergrößerung des Öffnungsquerschnitts mehr erfolgt.

Durch die Erfindung wird das Verformungsverhalten des Schlauchs begünstigt und kriechende Querschnittsveränderungen im Anschluß an das Abquetschen des Schlauchs werden vermieden.

Im folgenden wird unter Bezugnahme auf die Zeichnungen ein Ausführungsbeispiel der Erfindung näher erläutert.

Es zeigen:

Fig. 1 einen Längsschnitt durch die Schlauchklemme entlang der Linie I-I von Fig. 2, wobei der Schlauch und die Rolle gestrichelt dargestellt sind,

Fig. 2 einen Querschnitt durch die Schlauchklemme entlang der Linie II-II von Fig. 1,

Fig. 3 eine perspektivische Ansicht des Kanals gemäß einem Schnitt entlang der Linie III-III von Fig. 1, und

Fig. 4 in gleicher Darstellung wie Fig. 3 eine andere Ausbildung des Kanals.

Die Schlauchklemme weist ein langgestrecktes Gehäuse mit U-förmigen Querschnitt auf, das an der Oberseite offen ist. Die Seitenwände 11,12 sind an ihren unteren Enden durch die Bodenwand 13 verbunden. An den Innenseiten der Seitenwände 11,12 befinden sich parallele Führungsnuten 14 zur Führung der Rolle 15. Die Rolle 15 weist nach entgegengesetzten Seiten abstehende Achsstummel 16 auf, die in die Führungsnuten 14 hineinragen. Die Achsstummel 16 stehen von der Mittelscheibe 17 ab, die außen von einem verbreiterten Umfangsring 18 begrenzt wird. Die Umfangsfläche des Umfagsringes 18 ist mit einer Riffelung 19 versehen. Die Seitenwände 11 und 12 bilden zusammen mit der Bodenwand 13 einen längslaufenden Kanal 20, in dem die Rolle 15 in Längsrichtung bewegbar ist und aus dessen offener Oberseite die Rolle herausragt. Durch den Kanal 20 führt in Längsrichtung der Kunstoffschlauch 21 hindurch. Der Kanalboden 22 verläuft parallel zu den Führungsnuten 14.

Im Kanalboden 22 verläuft in der Mitte der Kanalbreite eine Ausweichnut 25, die am Freigabeende 23 die größte Tiefe und Breite und am Abquetschende 24 die geringste Tiefe und Breite hat. In dem Bereich 25a in der Nähe des Freigabeendes 23 sind Breite und Tiefe der Ausweichnut 25 konstant, während sich Breite und Tiefe der Auswreichnut im Bereich 25b, der den Regulierungsbereich bildet, linear zum Abquetschende 24 hin verringern.

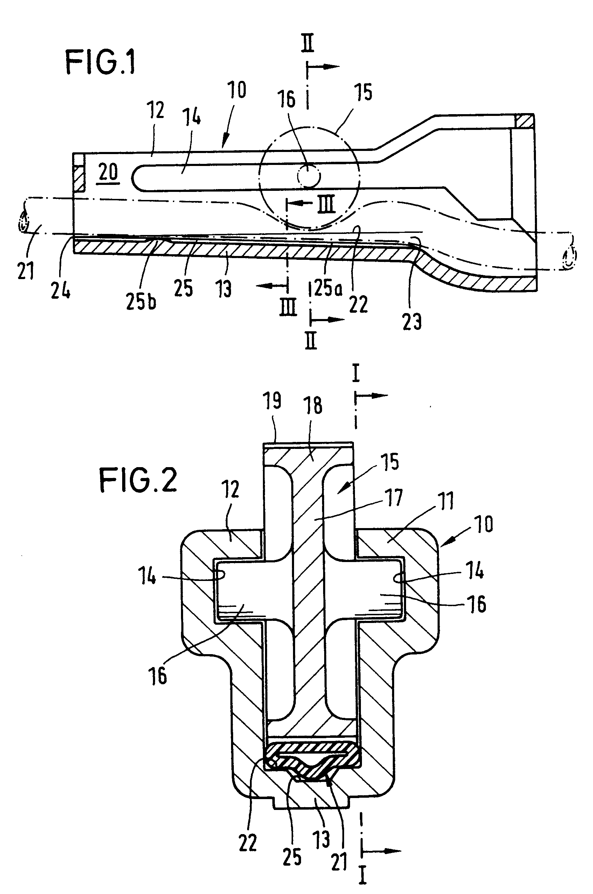

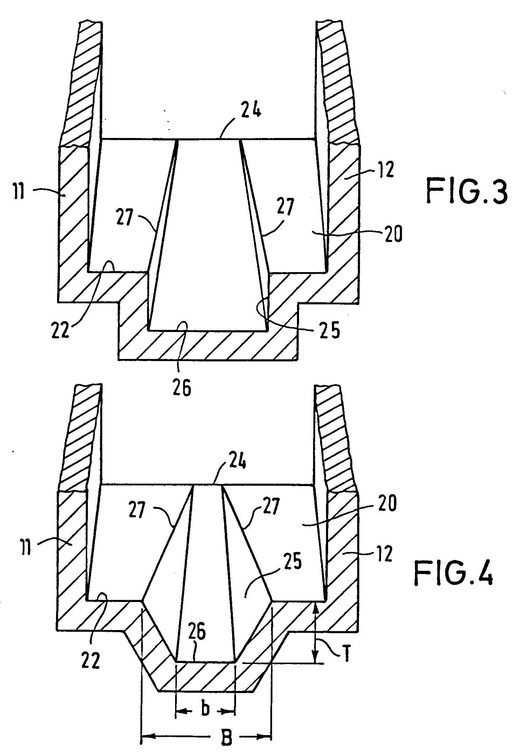

Bei dem Ausführungsbeispiel von Fig. 3 hat die Ausweichnut 25 rechteckigen Querschnitt. Die Breite der Ausweichnut 25 beträgt am breitesten Ende etwa die Hälfte der Kanalbreite und sie verringert sich zum Abquetschende 24 hin auf etwa ein Viertel der Kanalbreite. Die Tiefe der Ausweichnut 25 ist am breitesten Ende etwa halb so groß wie die Breite der Ausweichnut 25 und sie verringert sich zum Abquetschende 24 hin stetig bis auf Null. Der Boden 26 der Ausweichnut 25 bildet eine ebene Stützfläche, deren Abstand vom Kanalboden 22 sich zum Abquetschende 24 hin verringert. Wie aus Fig. 2 hervorgeht, ist die Breite des Schlauchs 21 im abgequetschten Zustand größer als die Breite des Kanals 22, so daß ein Teil des Schlauchquerschnitts in die Ausweichnut 25 verdrängt wird. Dieser verdrängte Teil des Schlauchquerschnitts wird von dem Boden 26 der Ausweichnut abgestützt. Die auf dem Kanalboden 22 aufliegenden Randbereiche des Schlauchs werden von der Rolle 15 vollständig abgequetscht, während der in die Ausweichnut 25 eingedrungene Bereich des Schlauchs einen Öffnungsquerschnitt freiläßt.

Bei dem Ausführungsbeispiel von Fig. 4 hat die Ausweichnut 25 trapezförmigen Querschnitt. Die Breite B der Ausweichnut ist am breitesten Ende etwa doppelt so groß wie die Breite b der Stützfläche 26. Zum Abquetschende 24 hin verringert sich die Breite der Stützfläche 26 nur relativ geringfügig, während die Breite der Ausweichnut 25 sich bis auf die Breite der Stützfläche 26 verringert. Die Tiefe T der Ausweichnut 25 ist am breitesten Ende etwa so groß wie die halbe Nutbreite B und sie verringert sich zum Abquetschende hin stetig auf Null. Durch die schrägen Seitenwände der Ausweichnut 25 wird der ausweichende Bereich des Schlauchquerschnitts auch seitlich abgestützt.

Bei allen beschriebenen Ausführungsbeispielen sind die Übergänge 27 zwischen Kanalboden 22 und Auswreichnut 25 scharfkantig, so daß eine deutliche Trennung der abgequetschten seitenbereiche des Schlauchs von dem den Öffnungsquerschnitt bildenden ausweichenden Mittelbereich erfolgt.

## Patentansprüche

1. Schlauchklemme für einen Kunststoffschlauch, mit einem in einem Gehäuse (10) ausgebildeten längslaufenden Kanal (20) für den Schlauch (21) und einer Rolle (15), deren Achse in Führungsnuten (14) der Seitenwände (11,12) des Kanals (20) geführt ist, wobei der Kanalboden (22) eine zentrisch angeordnete Ausweichnut (25) aufweist, deren Tiefe und Breite sich in Gehäuselängsrichtung zum Abquetschende (24) hin verringern, und die eine Stützfläche (26) für den ausweichenden Teil des Schlauchquerschnitts bildet, **dadurch gekennzeichnet** , daß die Ausweichnut (25) als Stützfläche (26) einen ebenen Boden aufweist, und daß am breitesten Ende der Ausweichnut die Breite (b) der Stützfläche (26) mindestens etwa die Hälfte der Nutbreite (B) und die Tiefe (T) der Ausweichnut etwa die Größe der halben Nutbreite (B) beträgt.

2. Schlauchklemme nach Anspruch 1, dadurch gekennzeichnet, daß die Ausweichnut (25) rechteckigen Querschnitt hat.

3. Schlauchklemme nach Anspruch 1, dadurch gekennzeichnet, daß die Ausweichnut (25) trapezförmigen Querschnitt hat.

4. Schlauchklemme nach einem der Ansprüche 1-3, dadurch gekennzeichnet, daß die Breite (b) der Stützfläche (26) am Abquetschende (24) mindestens ein Fünftel der Breite des Kanals (20) beträgt.

5. Schlauchklemme nach einem der Ansprüche 1-4, dadurch gekennzeichnet, daß der Übergang (27) zwischen Kanalboden (22) und Ausweichnut (25) scharfkantig ist.

## Revendications

1. Dispositif de serrage de tuyau souple pour un tuyau souple en matière synthétique comportant un canal (20) constitué longitudinalement dans un boîtier (10) pour le tuyau souple (21) et un galet (15), dont l'axe est guidé dans des rainures de guidage (14) des parois latérales (11, 12) du canal (20), où le fond du canal (22) présente une rainure d'expansion disposée au centre (25), dont la profondeur et la largeur vont en diminuant dans le sens longitudinal du logement en direction de l'extrémité de pincement (24), et qui forme une surface d'appui (26) pour la partie expansée de la section transversale du tuyau souple, caractérisée en ce que la rainure d'expansion (25) présente comme surface d'appui (26) un fond plan et en ce qu'à l'extrémité la plus large de la rainure d'expansion la largeur (b) de la surface d'appui (26) vaut au moins la moitié environ de la largeur (B) de la rainure et la profondeur (T) de la rainure d'expansion vaut environ la moitié de la largeur (B) de la rainure.

2. Dispositif de serrage de tuyau souple selon la revendication 1, caractérisé par le fait que la rainure d'expansion (25) a une section transversale rectangulaire.

3. Dispositif de serrage de tuyau souple selon la revendication 1, caractérisé par le fait que la rainure d'expansion (25) a une section transversale trapézoïdale.

4. Dispositif de serrage de tuyau souple selon l'une des revendications 1 à 3, caractérisé par le fait que la largeur (b) de la surface d'appui (26) atteint, à l'extrémité de pincement (24), au moins un cinquième de la largeur du canal (20).

5. Dispositif de serrage de tuyau souple selon l'une des revendications 1 à 4, caractérisé par le fait que la jonction (27) entre le fond (22) du canal et la rainure d'expansion (25) est à arêtes vives.

**Claims**

1. Tube clip for a plastics tube, having a longitudinally extending channel (20) for accomodating said tube (21), said channel being provided in a housing (10), and a roller (15), the axle of which is guided in guiding grooves (14) provided in the side walls (11, 12) of said channel (20), the channel bottom (22) being provided with a central evasion groove (25), the depth and width of which decrease in the longitudinal direction of the housing toward the squeeze end (24), and which form a support surface (26) for the evading portion of the tube cross section, **characterised in that** said evasion groove (25) has a plane bottom that provides a support surface (26), and that, at the widest end of said evasion groove, the width (b) of said support surface (26) is at least about half the groove width (B) and the depth (T) of said evasion groove is about half the groove width (B).

2. Tube clip according to claim 1, characterised in that said evasion groove (25) has a rectangular cross section.

3. Tube clip according to claim 1, characterised in that the evasion groove (25) has a trapezoidal cross section.

4. Tube clip according to one of claims 1-3, characterised in that the width (b) of said support surface (26) at the squeeze end (24) is at least one fifth of the width of said channel (20).

5. Tube clip according to one of claims 1-4, characterised in that the transition (27) between said channel bottom (22) and said evasion groove (25) has sharp edges.

FIG.1

FIG.2

FIG.3

FIG.4